# EUROPEAN PATENT APPLICATION

(11) **EP 2 377 538 A1**
(43) Date of publication of application: **19.10.2011**
(21) Application number: 08878873.2
(22) Date of filing: 17.12.2008
(51) Int. Cl.: A61K 31/4422, A61K 9/08, A61K 47/02, A61K 47/10, A61K 47/12, A61K 47/20, A61K 47/26, A61K 47/36, A61K 47/38

(54) **AQUEOUS ORAL PREPARATION OF STABLE AMLODIPINE**

(71) Applicant: Medrx Co., Ltd., Kagawa 769-2712 (JP)
(72) Inventor: TATSUMI, Noboru, Higashikagawa-shi Kagawa 769-2712 (JP); ENDO, Mitsuru, Higashikagawa-shi Kagawa 769-2712 (JP); HAMAMOTO, Hidetoshi, Higashikagawa-shi Kagawa 769-2712 (JP)
(74) Representative: Duckworth, Timothy John
(86) International application number: PCT/JP2008/003801
(87) International publication number: WO 2010/070705

(57) **Abstract**

The present invention aims to provide a stable aqueous oral preparation (liquid or jelly preparation) of amlosipine.

The object aqueous oral preparation (jelly preparation) of amlodipine, which is stable and highly rapidly disintegratable, could be produced by using an anionic surfactant having a sulfuric acid group or a sulfonic acid group as a stabilizer in an aqueous solution of amlodipine, preparing a liquid stable in the range of pH 5 - 7 as the liquid property, and adding and mixing a gelling agent, a fine powder solid and a gelling regulator. As a result, a novel oral preparation (liquid or jelly preparation) easy to take for older patients, who have declined swallowing function and face difficulty in taking tablets, can be provided.

## Description

### Technical Field

The present invention relates to a stable aqueous oral preparation of amlodipine, which contains an anionic surfactant having a sulfuric acid group or a sulfonic acid group. More particularly, the present invention relates to a jelly preparation of amlodipine, which is rapidly disintegratable and stable.

### Background Art

Amlodipine is described in US Patent No. 4,572,909, JP-AS58-167569 and the like, and has a chemical name of 2-(2-aminoethoxymethyl)-4-(2-chlorophenyl)-3-ethoxycarbonyl-5-methoxycarbonyl-6-methyl-1,4-dihydropyridine. Amlodipine is widely used in the world as a calcium channel antagonist (calcium-channel-blocker) that acts in the human body for a long time, and as a therapeutic agent for cardiovascular diseases such as angina pectoris, hypertension, congestive cardiac paralysis and the like.
Amlodipine itself is not satisfactory in photostability and preservation stability, and various salts are formed to improve them. For example, patent document 1 teaches that besylate salt and maleate salt are preferable from among various salts of amlodipine. Thus, amlodipine currently commercially available with the trade name of norvasc is a besylate salt.

However, since the besylate salt is still insufficient in photostability, an improvement in the preservation stability has been desired. In patent documents 2 and 3, therefore, ethylsulfonate and camphorsulfonate were prepared, using which the photostability and preservation stability were improved. In patent document 4, a stabilized preparation of a combination agent with alkali metal hydroxide is produced, where alkali metal hydroxide is added in an amount that adjusts an aqueous solution or dispersion thereof to pH 8. As a result, it is disclosed, the photostability is improved, and superior preservation stability along with prompt intestinal absorbability are exhibited.
In the efforts made to date, however, no disclosure relating to a liquid of amlodipine or a jelly preparation of amlodipine is available, and an attempt relating to a stable solution preparation or jelly preparation of amlodin is scarcely known heretofore.

patent document 1: EP89167
patent document 2: National Publication of International
Patent Application No. 2004-534008
patent document 3: National Publication of International
Patent Application No. 2006-500334
patent document 4: JP-A-2001-354565

### Disclosure of the Invention

### Problems to be Solved by the Invention

The present invention aims to provide a stable aqueous oral preparation of amlodipine (liquid and jelly preparation). Means of Solving the Problems

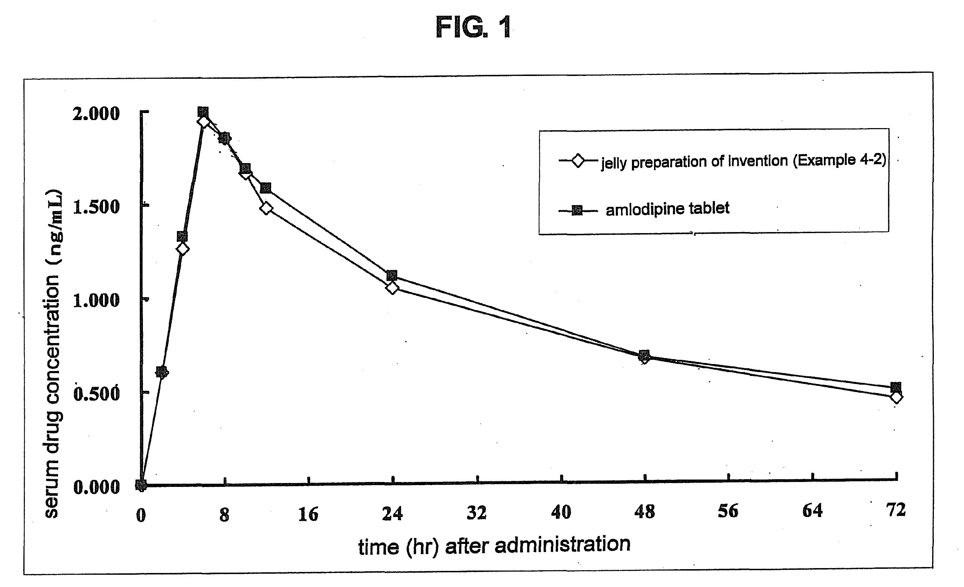

The present inventors have made intensive studies to produce a new aqueous oral preparation (liquid or jelly preparation) to be in place of the currently commercially available amlodipine·besylate tablets. As a result, they have found that the stability of amlodipine in a solution state can be improved by adding, as a stabilizer of amlodipine, an anionic surfactant having a sulfuric acid group or a sulfonic acid group. In addition, they have found that the bitter taste of amlodipine can also be masked by the addition of the anionic surfactant. As a result, they could complete a new aqueous oral preparation (liquid or jelly preparation), which is the object of the present invention.
Particularly, they have found that the jelly preparation of the present invention can be a rapidly disintegratable jelly preparation by adding a fine powder solid together with a gelling agent and mixing them. In addition, they have found that the strength of gel-matrix of the jelly preparation shows a bell-shaped curve depending on the concentration of a potassium salt etc. of organic acid or inorganic acid. Furthermore, an appropriate selection of these factors has enabled rapid disintegration of the gel-matrix in the stomach after oral to allow release of amlidipine. Consequently, the jelly preparation of the present invention achieves blood concentration that changes similarly to that of tablets, as shown in Fig. 1, thus exhibiting equivalency to tablets.

Accordingly, the gist of the present invention is as follows.
(1) A stable aqueous oral preparation of amlodipine, comprising 0.1 - 1 wt% of amlodipine, 0.2 - 2.5 wt% of a surfactant having a sulfuric acid group or a sulfonic acid group, and water, and showing a liquid property of pH 4.5 - 7.5.
(2) The aqueous oral amlodipine preparation of the above-mentioned (1), wherein the surfactant having a sulfuric acid group is sodium lauryl sulfate or sodium dodecyl sulfate.
(3) The aqueous oral amlodipine preparation of the above-mentioned 1 or 2, wherein the surfactant having a sulfuric acid group is sodium lauryl sulfate.
(4) The aqueous oral amlodipine preparation of any of the above-mentioned (1) - (3), further comprising an organic acid or a phosphoric acid.
(5) The aqueous oral amlodipine preparation of any of the above-mentioned (1) - (4), wherein the organic acid is one or more selected from citric acid, acetic acid, malic acid and tartaric acid.
(6) The aqueous oral amlodipine preparation of any of the above-mentioned (1) - (5), further comprising 15 - 45 wt% of a sweetener.
(7) The aqueous oral amlodipine preparation of any of the above-mentioned (1) - (6), wherein the sweetener is sugar and/or sugar alcohol.
(8) The aqueous oral amlodipine preparation of any of the above-mentioned (1) - (7), wherein the sugar is one or more selected from sucrose, fructose, hydrogenated maltose sugar and sucralose, and the sugar alcohol is one or more selected from sorbitol, mannitol and erythritol.
(9) The aqueous oral amlodipine preparation of any of the above-mentioned (1) - (8), further comprising a nonionic surfactant.
(10) The aqueous oral amlodipine preparation of any of the above-mentioned (9), wherein the nonionic surfactant is a sucrose ester of fatty acid.
(11) The aqueous oral amlodipine preparation of any of the above-mentioned (1) - (10), further comprising menthol as a flavor.
(12) The aqueous oral amlodipine preparation of any of the above-mentioned (1) - (11), which is a liquid.

(13) The aqueous oral amlodipine preparation of any of the above-mentioned (1) - (11), further comprising 0.1 - 3 wt% of a gelling agent and 0.2 - 5 parts by weight of a fine powder solid.
(14) The aqueous oral amlodipine preparation of any of the above-mentioned (1) - (12), further comprising 0.1 - 2 wt% of a gelling regulator.
(15) The aqueous oral amlodipine preparation of the above-mentioned (14), wherein the gelling regulator is a potassium salt compound.
(16) The aqueous oral amlodipine preparation of the above-mentioned (15), wherein the potassium salt compound is potassium chloride.
(17) The aqueous oral amlodipine preparation of any of the above-mentioned (13) - (16), wherein the gelling agent is carageenan.
(18) The aqueous oral amlodipine preparation of any of the above-mentioned (13) - (17), wherein the fine powder solid is one or more selected from a cellulose derivative, light anhydrous silicic acid and talc.
(19) The aqueous oral amlodipine preparation of the above-mentioned (18), wherein the cellulose derivative is crystalline cellulose.
(20) The aqueous oral amlodipine preparation of the above-mentioned (13) - (19), which is a jelly preparation.

(21) A stable jelly preparation of amlodipine of the above-mentioned (19) having the following composition:
   a) 0.1 - 1 wt% of amlodipine,
   b) 0.2 - 2.5 wt% of an anionic surfactant having a sulfuric acid group or a sulfonic acid group,
   c) 0.1 - 3 wt% of a gelling agent,
   d) 0.1 - 2 wt% of a gelling regulator,
   e) 0.2 - 3 wt% of a fine powder solid.
(22) The stable jelly preparation of the above-mentioned (20) having a liquid property of pH 5 - 7, wherein the composition comprises the following:
   a) 0.1 - 1 wt% of amlodipine,
   b) sodium lauryl sulfate as the anionic surfactant having a sulfuric acid group or a sulfonic acid group,
   c) carageenan as the gelling agent,
   d) potassium chloride as the gelling regulator,
   e) crystalline cellulose and light anhydrous silicic acid as the fine powder solid,
   f) sorbitol as the sweetener.
(23) The stable jelly preparation of the above-mentioned (20) having a liquid property of pH 5 - 7, wherein the composition comprises the following:
   a) 0.1 - 1 wt% of amlodipine,
   b) 0.2 - 2.0 wt% of sodium lauryl sulfate,
   c) 0.5 - 2 wt% of carageenan,
   d) 0.2 - 1 wt% of potassium chloride,
   e) 0.5 - 2 wt% of crystalline cellulose and light anhydrous silicic acid,
   f) 20 - 45 wt% of sorbitol.
(24) The stable jelly preparation of the above-mentioned (20) - (23), further comprising 0.05 - 2.0 wt% of a nonionic surfactant.
(25) The stable jelly preparation of the above-mentioned (20) - (24), wherein the nonionic surfactant is a sucrose ester of fatty acid.

### Effect of the Invention

In the novel aqueous oral preparation (liquid or jelly preparation) of the present invention, an anionic surfactant such as sodium lauryl sulfate and the like is used, which enables stabilization of amlodipine·besylate and masking of a bitter taste. As a result, an aqueous oral preparation with a dosage form (liquid or jelly preparation) different from tablets and very easy to take can be provided. Generally, tablet intake often causes difficulty for older patients showing decline in swallowing function. However, the novel aqueous oral preparation of the present invention can overcome such problem. Therefore, the aqueous oral preparation of the present invention can provide a novel amlodipine preparation highly convenient particularly for older patients.

### Best Description of Embodiments

The "amlodipine" in the context of the present invention refers to amlodipine·besylate, unless particularly specified. The concentration of amlodipine·besylate in the preparation of the present invention (liquid or jelly preparation) is 0.1 - 1 wt%, preferably 0.2 - 0.7 wt%, more preferably 0.3 - 0.5 wt%.
The "anionic surfactant having a sulfuric acid group or a sulfonic acid group" in the context of the present invention refers to orally administrable anionic surfactant having a sulfuric acid group or a sulfonic acid group. Examples of the surfactant having a sulfuric acid group include alkyl sulfate sodium (AS) such as lauryl sulfuric acid Na, dodecylsulfuric acid Na and the like, and alkylethersulfuric acid ester sodium (AES) such as polyoxyethylene alkylethersulfuric acid ester and the like. Examples of the anionic surfactant having a sulfonic acid group include straight chain alkylbenzenesulfonic acid sodium (LAS) or branched alkylbenzenesulfonic acid sodium (ABS) such as sodium dodecylbenzenesulfonate and the like, α-sulfo fatty acid ester sodium (ASF, TMS) such as dialkylsulfosuccinic acid Na and the like; α-olefin sulfonic acid compounds such as olefin(C14-16)sulfonic acid Na and the like, and alkylsulfonic acid compounds such as methanesulfonic acid Na, ethanesulfonic acid Na, camphorsulfonic acid Na and the like. Preferred are those having strong acidity and less toxicity, and more preferred are alkylsulfuric acid compounds such as sodium lauryl sulfate, sodium dodecylsulfate and the like.
The concentration of an anionic surfactant having a sulfuric acid group or a sulfonic acid group to be used for a liquid is, for example, 0.2 - 2.5%, preferably 0.2 - 1.5 wt%, more preferably 0.3 - 0.8 wt%. In addition, when the concentration of an anionic surfactant having a sulfuric acid group or a sulfonic acid group to be used is not less than 2-fold the molar amount of amlodipine in the preparation, good effects can be obtained.

The "water" in the context of the present invention refers to orally usable purified water. The water is not particularly limited as long as it can be used for the production of pharmaceutical products.
The "organic acid" in the context of the present invention generally refers to organic acids and alkali metal salts thereof, which can be used for pharmaceutical products. For example, citric acid, sodium citrate, disodium citrate, succinic acid, acetic acid, tartaric acid, D-tartaric acid, potassium hydrogen tartrate, sodium DL-tartrate, lactic acid, sodium lactate, fumaric acid, monosodium fumarate, maleic acid, dl-malic acid, sodium dl-malate, phosphoric acid, trisodium phosphate, sodium hydrogenphosphate, dipotassium phosphate, potassium dihydrogen phosphate, sodium dihydrogen phosphate and the like can be mentioned. Preferred are citric acid, sodium citrate, disodium citrate, dl-malic acid, sodium dl-malate, phosphoric acid, trisodium phosphate, sodium hydrogenphosphate, dipotassium phosphate, potassium dihydrogen phosphate and sodium dihydrogen phosphate, and particularly preferred are citric acid, sodium citrate and disodium citrate.
Organic acids can provide, along with a bitter taste masking action of amlodipine, a pH buffering action when combined with a sodium salt thereof. Therefore, organic acids can be used as a bitter taste masking agent, a pH regulator or a pH buffering agent. The concentration of the organic acid to be used is generally desirably not less than the concentration at which the bitter taste can be masked or pH can be buffered. That is, the concentration of organic acid and organic acid sodium salt to be used is, for example, within the range of 1 - 5 wt%, preferably 1.5 - 4.0 wt%.
When, for example, citric acid is used in the present invention, masking of the bitter taste of amlodipine with citric acid alone is difficult, and addition of an anionic surfactant such as sodium lauryl sulfate is indispensable. The results have clarified that an anionic surfactant such as sodium lauryl sulfate significantly contributes to the masking of a bitter taste. In the preparation of the present invention, citric acid is considered to greatly contribute mainly to a pH adjusting or buffering action, even though it is slightly involved in the masking of a bitter taste. In the preparation of the present invention, the liquid property needs to be adjusted to pH 4.5 - 7.5, and citric acid is desirably used in an amount capable of adjusting pH to this range. The amount thereof to be used is, for example, the range of 1 - 5 wt% similar to the above-mentioned range. Preferred is the range of 1.5 - 4.0 wt%.

The "liquid property" in the context of the present invention refers to the pH of an aqueous solution preparation (to be referred to as gel-base) before addition of a gelling agent and a fine powder solid, or the pH of a jelly preparation formed after addition of a gelling agent and a fine powder solid. pH can be measured by using a general measurement means such as pH test paper, pH meter and the like. For example, pH of an aqueous solution preparation can be measured using a stock solution. In the case of a jelly preparation, pH can be directly measured by a measurement means such as pH test paper, pH meter and the like, or can be measured by separating a few grams of a jelly composition, diluting the composition with 10- to 100-fold volume of purified water, and measuring the solution with a pH meter.
The liquid property of the liquid or jelly preparation of the present invention is preferably not more than pH 7.5, preferably not less than pH 4.5. More preferably, it is not less than pH 5.
When the pH is not less than 5, the water separation tendency of a jelly preparation can be further suppressed. Therefore, more preferable pH of a jelly preparation is pH 5 - 7.

The "sweetener" in the context of the present invention is not particularly limited as long as it is used to add sweetness to a preparation. The "sugar" in the present specification refers, but is not particularly limited, to sucrose, lactose, glucose, liquid sugar, fructose, fructose glucose liquid sugar, caramel, hydrogenated maltose syrup, brown sugar, sucrose, simple syrup, powder sugar, starch syrup, maltose, powder candy and the like. They can be used regardless of the kind of the sugar. While the "sugar alcohol" refers to sorbitol, mannitol, xylitol, glycerol, erythritol and the like, it is not particularly limited and can be used regardless of the kind of the sugar alcohol. Furthermore, some sugars and sugar alcohols can be used in a mixture. Preferable examples of the sweetener include sucrose, reduced malt sugar starch syrup, sorbitol, mannitol, glucose and saccharin sodium. More preferable examples include hydrogenated maltose syrup, sucralose, sorbitol and mannitol.
The concentration of the sweetener to be used is desirably set such that the characteristic bitter taste of amlodipine would be masked when used together with an organic acid, thus facilitating the ingestion. When sugar alcohol is used, a higher concentration thereof tends to improve disintegration property of a jelly preparation (releasing ability of amlodipine). For example, when sorbitol is used as sugar alcohol, the disintegration property of the jelly preparation is superior at about 25 wt% to that at about 18 wt%, and the disintegration property is further improved at 30 wt%.
As mentioned above, the sweetener of the present invention can promote disintegration property of a jelly preparation depending on the concentration to be employed. Therefore, in consideration of the control of the amlodipine releasing property, the concentration to be used is within the range of 15 - 50 wt%. Preferred is the range of 20 - 45 wt%, and more preferred is the range of 25 - 45 wt%. On the other hand, the results of sensory evaluation of the preparation reveal that too strong sweetness does not provide comfortable feeling during use. In view of the sensory evaluation, therefore, the concentration of the sweetener to be used is considered to be preferable at not more than 40 wt%.

The "nonionic surfactant" in the context of the present invention refers to a nonionic surfactant permitting oral administration. Examples thereof include sucrose ester of fatty acid which is an ester form of stearic acid, palmitic acid, myristic acid, oleic acid, lauric acid and the like, and a hydroxy group of sucrose, sorbitan ester of fatty acid such as sorbitan monostearate, sorbitan monooleate, sorbitan cocoate and the like, glycerol esters of fatty acid such as acetic acid monoglyceride, lactic acid monoglyceride, citric acid monoglyceride, diacetyl tartaric acid monoglyceride, succinic acid monoglyceride, polyglycerol ester of fatty acid, polyglycerol esters of interesterified rinocileic acid and the like, hydrogenated castor oil and the like. Preferred is sucrose ester of fatty acid.
The concentration of the nonionic surfactant to be used is sufficient as long as it is within the range of 0.05 - 2.0 wt%. Preferred is the range of 0.05 - 1.0 wt%, more preferred is the range of 0.05 - 0.5 wt%.

In the present invention, pH adjuster, antioxidant, preservative, flavor and the like can also be added as appropriate according to the object.
Examples of the pH adjuster include buffering agents comprising an organic acid such as citric acid, tartaric acid, lactic acid, fumaric acid, malic acid and the like and an alkali metal salt thereof, and buffering agents comprising an inorganic acid such as phosphoric acid and the like and an alkali metal salt thereof. The liquid property of the liquid or jelly preparation of the present invention is preferably adjusted to the range of not more than pH 7.5, more preferably not less than pH 4.5. Still more preferred is the range of pH 5 - 7.
Examples of the antioxidant include ascorbic acid, sodium bisulfite, sodium sulfite, erythorbic acid, tocopherol acetate, dibutylhydroxytoluene, tocopherol, sodium pyrrosulfite, butylhydroxyanisole, propyl gallate and the like.

Examples of the preservative include benzoic acid, sodium benzoate, sorbic acid, sodium sorbate, sodium dehydroacetate, p-hydroxybenzoic acid, sodium p-hydroxybenzoate, ethyl p-hydroxybenzoate, propyl p-hydroxybenzoate(propylparaben), butyl p-hydroxybenzoate, isopropyl p-hydroxybenzoate, isobutyl p-hydroxybenzoate, propionic acid, sodium propionate and the like.
To uniformly dissolve these additives, a solubilizing agent can be appropriately used. The solubilizing agent to be used here means water or a hydrophilic oily solvent. For example, polyhydric alcohols such as concentrated glycerol, glycerol, polyvinyl alcohol, propylene glycol, ethylene glycol and the like can be mentioned. Preferred are concentrated glycerol and polyvinyl alcohol.
The flavor is not particularly limited as long as it is used for addition of a flavor. For example, menthol, peppermint oil, vanilla essence, coffee flavor and the like can be mentioned. Preferred are menthol and peppermint oil for addition of a refreshing flavor.

Examples of the "gelling agent" in the context of the present invention include gum arabic, gum arabic powder, alginic acid, sodium alginate, alginic acid propyleneglycol ester, carageenan, karaya gum powder, carob bean gum, curdlan, agar, agar powder, xanthan gum, guar gum, psyllium seed gum, gellan gum, purified gelatin, gelatin, tamarind seed gum, tara gum, tragacanth, tragacanth powder, furcelleran, pullulan, pectin and the like, and the agent may be a mixture thereof. Preferable examples thereof include sodium alginate, carageenan, carob bean gum, agar powder, xanthan gum and pectin. Particularly preferred are carageenan and carob bean gum. A jelly preparation containing carageenan is preferably set to liquid property of pH 4. - 7.5.
In general, carageenan is not gelled. However, gelling can be promoted by increasing the viscosity, whereby good gel property and disintegratability can be obtained.
The concentration of the gelling agent to be used is sufficient when it is within the range of 0.1 - 3 wt%, preferably 0.5 - 2 wt%, more preferably 1 - 2 wt%.

The "fine powder solid" in the context of the present invention refers to a hardly water-soluble substance in the form of a fine powder and a solid at ambient temperature. The fine powder means a powder having a particle size of 1 - 500 µm, desirably a finer particle size. The hardly water-soluble substance which is solid at ambient temperature refers to a hardly water-soluble or insoluble inorganic material, or a hardly water-soluble or insoluble organic material. While the solubility of these materials varies depending on the liquid property forming a jelly composition, unless particularly specified, it means the solubility of neutral water. Being hardly water-soluble generally means being hardly soluble in water. For example, a solubility of not more than 3 w/w% (amount of solute dissolved in cold water (100 g) is not more than 3 g) is "hardly water-soluble". Preferably, it is not more than 1 w/w%.
Among the hardly water-soluble substances which are solid at ambient temperature, examples of inorganic materials include non-crystalline silicon dioxide, kaolin (gypsum), diatomaceous earth, talc, hydrated silicon dioxide, light anhydrous silicic acid, magnesium silicate, calcium silicate, calcium phosphate, barium sulfate and the like. Examples of the organic material include crystalline cellulose, starch, activated carbon, hardly soluble organic low-molecular-weight compound and the like.
The concentration of the fine powder solid to be used is sufficient when it is within the range of 0.2 - 5 wt%, preferably 0.5 - 3 wt%, more preferably 0.5 - 2.0 wt%.

The "gelling regulator" in the context of the present invention refers to one used for controlling the strength of a gel-matrix constructed with a gelling agent, and refers to a potassium salt or an alkaline earth metal salt of an organic acid or an inorganic acid. When the amount of the gelling regulator increases, a gel having a strong strength is obtained; however, when the amount exceeds a given level, the gel strength conversely becomes weak. Therefore, with the concentration of the gelling regulator on the horizontal axis and the gel strength on the vertical axis, a bell-shaped strength curve is depicted.
Examples of the organic acid as the gelling regulator include acetic acid, oxalic acid, citric acid, malic acid, lactic acid and the like. Examples of the inorganic acid as the gelling regulator include phosphoric acid, hydrochloric acid, sulfuric acid and the like. In addition, examples of the alkaline earth metal include magnesium, calcium, aluminum and the like.
When a water-soluble potassium salt is used, for example, potassium chloride, potassium acetate, potassium oxalate and the like can be used.
Thus, as the potassium salt and alkaline earth metal salt usable in the present invention, one or more kinds may be selected from the above and used in combination.
Preferable examples of the potassium salt to be used in the present invention include potassium chloride, potassium citrate and the like, and examples of the alkaline earth metal salt include organic acid calcium salt such as calcium citrate and the like.
The concentration of the gelling regulator to be used is sufficient when it is within the range of 0.1 - 2 wt%, preferably 0.2 - 1.5 wt%, more preferably 0.2 - 1.0 wt%.

In the present invention, the liquid property of the liquid (gel-base) before addition of a gelling agent and a fine powder solid is not more than pH 7. The liquid property of a jelly composition formed after addition of a gelling agent and a fine powder solid is preferably within the pH range of about 4.5 - 7.5. The liquid property of a gel-base and the like can be measured by a conventional liquid property measurement device such as pH meter and the like, and can be adjusted to satisfy the above-mentioned liquid property. Particularly, when the liquid property of a liquid (gel-base) containing an efficacy component dissolved therein is near neutral, the efficacy component is generally considered to be stable. Therefore, it is preferable to adjust the amount ratio of the reagents to be added such that the liquid property of the gel-base containing amlodipine dissolved therein and that of the final gel composition would be pH 5 - 7 or neutral.

The present invention is explained in more detail in the following by referring to Examples, which are not to be construed as limitative. The present invention can also be practiced on appropriate modification without departing from the above- and below-mentioned gist, and all of them are encompassed in the technical scope of the present invention. Examples

### (Example 1) Preparation of amlodipine liquid

Respective reagents were weighed according to the composition in Table 1 below (numerical values in the Table are in wt%).
To water were added D sorbitol solution, citric acid, sodium citrate and sucralose, and the mixture was stirred with heating at 60°C. Then, amlodipine, sucrose ester of fatty acid and propylparaben were added to give an amlodipine solution.
The result thereof is shown together in Table 1.

**Table 1**

| | |
|---|---|
| amlodipine besylate | 0.347 |
| sodium lauryl sulfate | 0.4 |
| sucrose ester of fatty acid | 0.1 |
| citric acid | 0.6 |
| sodium citrate | 1.7 |
| D sorbitol | 18 |
| sucralose | 0.1 |
| propylparaben | 0.04 |
| water | balance |
| total | 100 |

### (Experimental Example 1) Preservation stability of amlodipine liquid

### (Comparison test between the presence and absence of coexisting anionic surfactant·sodium lauryl sulfate)

The liquid prepared in Example 1 was preserved in a 80°C incubator for 24 hr, and the preservation stability of amlodipine was examined. As a result, good preservation stability was obtained in the presence of sodium lauryl sulfate; however, the preservation stability decreased by about 15% in the absence of sodium lauryl sulfate.

### (Example 2) Preparation of amlodipine jelly preparation

Respective reagents were weighed according to the composition in Table 2 below (numerical values in the Table are in wt%).
To water were sequentially added with heating light anhydrous silicic acid, crystalline cellulose, potassium chloride, D sorbitol, carageenan, citric acid, sodium citrate, sodium lauryl sulfate and sucralose. After stirring with heating at 80°C, amlodipine, sucrose ester of fatty acid and propylparaben were added, and the mixture was stirred with heating at 85°C as a sterilization step. The mixture was dispensed to a predetermined container while it was warm and allowed to cool naturally to give a jelly preparation.
The jelly preparation (1 g) was separated and diluted with purified water to a total amount of 20 g. The pH value of the liquid property was measured and found to be pH 5.3.

### (Reference Example 1) Preparation of a jelly preparation not containing anionic surfactant (sodium lauryl sulfate)

Respective reagents were weighed according to the composition in Table 2 below (numerical values in the Table are in wt%) to give a jelly preparation. First, to water were sequentially added with heating light anhydrous silicic acid, crystalline cellulose, potassium chloride, D sorbitol, carageenan, citric acid, sodium citrate and sucralose. After stirring with heating at 80°C, amlodipine, sucrose ester of fatty acid and propylparaben were added, and the mixture was stirred with heating at 85°C as a sterilization step. The mixture was dispensed to a predetermined container while it was warm and allowed to cool naturally to give a jelly preparation. In the same manner as in Example 1, the pH of the jelly preparation was measured and found to be pH 5.3.
The results of Example 2 and Reference Example 1 are described together in Table 2.

**Table 2**

| | Example 2 | Reference Example 1 |
|---|---|---|
| amlodipine besylate | 0.347 | 0.347 |
| sodium lauryl sulfate | 0.4 | 0 |
| sucrose ester of fatty acid | 0.1 | 0.1 |
| citric acid | 0.6 | 0.6 |
| sodium citrate | 1.7 | 1.7 |
| sucralose | 0.1 | 0.1 |
| D sorbitol powder | 17.5 | 17.5 |
| carageenan | 1.2 | 1.2 |
| potassium chloride | 0.6 | 0.6 |
| crystalline cellulose | 1 | 1 |
| light anhydrous silicic acid | 0.3 | 0.3 |
| propylparaben | 0.04 | 0.04 |
| water | balance | balance |
| total | 100 | 100 |

### (Experimental Example 2) Preservation stability of amlodipine jelly preparation

### (Comparison test between the presence and absence of anionic surfactant·sodium lauryl sulfate)

### (1) Preservation stability at 60°C

The jelly preparations produced in Example 2 and Reference Example 1 were preserved in a 60°C incubator for 1 week. Thereafter, the content of amlodipine was measured, and the preservation stability of amlodipine in the jelly preparations was examined. The results are shown in the following Table 3.

**Table 3**

| | Example 2 | Reference Example 1 |
|---|---|---|
| content of amlodipine in preparation (2 g) (after lapse of 1 week at 60°C) | 100% | 73% |

As shown in the above results, the presence of sodium lauryl sulfate in the jelly preparation was found to significantly stabilize amlodipine.

### (Experimental Example 3) Disintegration property of amlodipine jelly preparation

The jelly preparations produced in Example 2 (about 2 g) was separated and applied to a dissolution tester. First, the jelly preparation was immersed in the Japanese Pharmacopoeia disintegration test solution 1st fluid (pH 1.2) and 2nd fluid (pH 6.8). The dissolution ratio of amlodipine into each aqueous solution was measured 5 min, 15 min, 30 min and 60 min after addition of the jelly preparation.
The dissolution rate of the jelly preparation of Example 2 in each solution is shown in Table 4.

**[Table 4]**

| | 5 min later | 15 min later | 30 min later | 60 min later |
|---|---|---|---|---|
| Japanese Pharmacopoeia disintegration test solution 1st fluid | 38% | 71% | 82% | 84% |
| Japanese Pharmacopoeia test disintegration test solution 2nd fluid | 12% | 45% | 78% | 81% |

As shown in the above results, the jelly preparation of Example 2 was found to rapidly disintegrate in each solution of the Japanese Pharmacopoeia disintegration test solution 1st fluid and 2nd fluid to allow elution of amlodin in each solution.

### (Example 3) Improvement of dissolution property by increase of sugar content

In the same manner as in Example 2, a jelly preparation having the composition of Table 5 (numerical values in the Table are in wt%) except that the content of sorbitol was increased 1.4-fold was prepared. The pH of the jelly preparation was measured in the same manner as in Example 1 to find pH 5.3.

**[Table 5]**

| | Example 3 |
|---|---|
| amlodipine besylate | 0.347 |
| sodium lauryl sulfate | 0.4 |
| sucrose ester of fatty acid | 0.1 |
| citric acid | 0.6 |
| sodium citrate | 1.7 |
| sucralose | 0.1 |
| D sorbitol powder | 24.5 |
| carageenan | 1.2 |
| potassium chloride | 0.4 |
| crystalline cellulose | 1 |
| light anhydrous silicic acid | 0.3 |
| propylparaben | 0.04 |
| water | balance |
| total | 100 |

### (Experimental Example 4) Disintegration property of amlodipine jelly preparation

In the same manner as in Experimental Example 3, the disintegration property of the jelly preparations produced in Example 3 and the dissolution rate of amlidipine into each aqueous solution were measured.
The dissolution rates of the jelly preparation of Example 3 in each solution are shown in Table 6.

**[Table 6]**

| | 5 min later | 15 min later | 30 min later | 60 min later |
|---|---|---|---|---|
| Japanese Pharmacopoeia disintegration test solution 1st fluid | 73% | 84% | 92% | 94% |
| Japanese Pharmacopoeia disintegration test solution 2nd fluid | 54% | 90% | 96% | 98% |

From the above results, the dissolution property of amlodipine in the jelly preparation of Example 3 was improved by increasing the amount of sugar alcohol (D sorbitol) to be added.

### (Experimental Example 5) Preservation stability of amlodipine jelly preparation

### (1) Preservation stability at 40°C and 50°C

The jelly preparations produced in Example 3 was preserved in 40°C and 50°C incubators for 2 months. Thereafter, the content of amlodipine was measured, and the preservation stability of amlodipine in the jelly preparation was examined. The results are shown in the following Table 7.

**[Table 7]**

| | Example 3 |
|---|---|
| content of amlodipine in preparation (2 g, after lapse of 2 months at 40°C) | 99% |
| content of amlodipine in preparation (2 g, after lapse of 2 months at 50°C) | 96% |

From the above results, the jelly preparation of Example 3 was shown to also have good preservation stability.

### (Example 4) Influence of increased pH and increased anionic surfactant content on jelly preparation

To improve the jelly preparation of Example 3, neutralization of pH, and an increase of the contents of sugar and anionic surfactant were studied. In the same manner as in Example 2, respective reagents were weighed according to the composition of Table 8 (numerical values in the Table are in wt%) and jelly preparations were prepared (Example 4-1, Example 4-2, Example 4-3). Each jelly preparation (1 g) was separated and diluted with purified water to the total amount of 20 g, and the pH of the liquid property was measured. Furthermore, to evaluate water separation of the jelly preparations, appearance of the jelly preparations after preserving at room temperature for 1 day was visually observed. The results thereof are shown together in Table 8.

**[Table 8]**

| | Example 3 | Example 4-1 | Example 4-2 | Example 4-3 |
|---|---|---|---|---|
| amlodipine besylate | 0.347 | 0.347 | 0.347 | 0.347 |
| sodium lauryl sulfate | 0.4 | 0.7 | 0.7 | 0.7 |
| sucrose ester of fatty acid | 0.1 | 0.2 | 0.2 | 0.2 |
| citric acid | 0.6 | 0.37 | 0.28 | 0.115 |
| sodium citrate | 1.7 | 2 | 2.18 | 2.39 |
| sucralose | 0.1 | 0.12 | 0.12 | 0.12 |
| D sorbitol powder | 24.5 | 30 | 30 | 30 |
| carageenan | 1.2 | 1.2 | 1.2 | 1.2 |
| potassium chloride | 0.4 | 0.2 | 0.2 | 0.2 |
| crystalline cellulose | 1 | 1 | 1 | 1 |
| light anhydrous silicic acid | 0.3 | 0.3 | 0.3 | 0.3 |
| propylparaben | 0.04 | 0.04 | 0.04 | 0.04 |
| water | balance | balance | balance | balance |
| total | 100 | 100 | 100 | 100 |
| pH | 5.3 | 5.5 | 6.1 | 6.7 |
| water separation | slight water separation | slight water separation | no water separation | no water separation |

### (Experimental Example 6) Disintegration property of amlodipine jelly preparation

Of the jelly preparations produced in Example 4, Example 4-2 and Example 4-3 wherein the water separation was not confirmed were studied for the disintegration property, and the dissolution rates of amlodipine into aqueous solutions were measured.
The dissolution rate of the jelly preparation of Example 4-2 in each solution is shown in Table 9, and the results of Example 4-3 are shown in Table 10.

**[Table 9]**

| | 5 min later | 15 min later | 30 min later | 60 min later |
|---|---|---|---|---|
| Japanese Pharmacopoeia disintegration test solution 1st fluid | 49% | 74% | 93% | 99% |
| Japanese Pharmacopoeia disintegration test solution 2nd fluid | 11% | 37% | 65% | 98% |

**[Table 10]**

| | 5 min later | 15 min later | 30 min later | 60 min later |
|---|---|---|---|---|
| Japanese Pharmacopoeia disintegration test solution 1st fluid | 21% | 65% | 96% | 98% |
| Japanese Pharmacopoeia disintegration test solution 2nd fluid | 6% | 15% | 30% | 57% |

From the above-mentioned results, the jelly preparation of Example 4-2 was found to exhibit more superior disintegration property.

### (Experimental Example 7) Preservation stability of amlodipine jelly preparation

### Preservation stability at 25°C, 40°C and 50°C

The jelly preparation produced in Example 4-2 was preserved in 25°C, 40°C and 50°C incubators, and preserved at 25°C for 12 month, at 40°C for 6 month, and at 50°C for 3 month. The content of amlodipine was measured at appropriate timing, and the preservation stability of amlodipine in the jelly preparation was examined. The results are shown relative to initial % in the following Table 11.

**[Table 11]**

| | 1 | 2 | 3 | 6 | 9 | 12 |
|---|---|---|---|---|---|---|
| | month | months | months | months | months | months |
| 25°C | - | - | 99% | 99% | 98% | 98% |
| 40°C | 99% | 99% | 97% | 97% | - | - |
| 50°C | 97% | 96% | 95% | - | - | - |

From the above-mentioned results, the jelly preparation of Example 4-2 was found to completely disintegrate within 1 hr, elute rapidly into the Japanese Pharmacopoeia disintegration test solution 1st fluid and 2nd fluid as compared to Example 2 and Example 3, and be superior in thermal stability.

### (Experimental Example 8) Biological equivalency test of amlodipine (registered trade mark, 5 mg tablet) and jelly preparation of Example 4-2 (5 mg amlodipine/2 g jelly) in human

The jelly preparation (2 g) of Example 4-2 or amlodipine (registered trade mark, 5 mg tablet, manufactured and distributed by Dainippon Sumitomo Pharma Co., Ltd., 1 tablet) was orally administered once to 18 target healthy men under fasting according to the cross-over study at one agent one stage. The serum concentration of amlodipine was measured, and the biological equivalency of the both preparations was verified from the pharmacokinetics.

As a result, as shown in Fig. 1, the jelly preparation of Example 4-2 (amlodipine 5 mg) and amlodipine tablet (amlodipine (registered trade mark) tablet 5 mg) were found to be biological equivalent. As the evaluation results of the biological equivalency to the main evaluation items (AUC₀₋₇₁, Cₘₐₓ), the 90% confidence interval in the difference between medicaments and difference in the average values after logarithmic conversion were log (0.9685) and log (0.9016) - log (1.0404) in AUC₀₋₇₁, and log (1.0019) and log (0.9141) - log (1.0981) in Cₘₐₓ. All parameters were within the range of biological equivalency standard, log (0.80) - log (1.15).

### Industrial Applicability

In the present invention, an aqueous oral preparation of amlodipine (liquid or jelly preparation) could be produced by using amlodipine, which is not very stable in aqueous solutions, and finding an anionic surfactant such as sodium lauryl sulfate as a stabilizer. As a result, a preparation easy to take for older patients prone to accidental ingestion, instead of tablets which are difficult to swallow, could be provided.

### Brief Description of the Drawing

Fig. 1 shows changes in the serum amlodipine concentrations after ingestion of the preparation (jelly preparation) of the present invention and a tablet.

## Claims

1. A stable aqueous oral preparation of amlodipine, comprising 0.1 - 1 wt% of amlodipine, 0.2 - 2.5 wt% of an anionic surfactant having a sulfuric acid group or a sulfonic acid group, and water, and showing a liquid property of pH 4.5 - 7.5.

2. The aqueous oral amlodipine preparation of claim 1, wherein the anionic surfactant having a sulfuric acid group is sodium lauryl sulfate.

3. The aqueous oral amlodipine preparation of claim 1 or 2, further comprising a nonionic surfactant.

4. The aqueous oral amlodipine preparation of any of claims 1 to 3, wherein the nonionic surfactant is a sucrose ester of fatty acid.

5. The aqueous oral amlodipine preparation of any of claims 1 to 4, further comprising an organic acid.

6. The aqueous oral amlodipine preparation of any of claim 5, wherein the organic acid is one or more selected from citric acid and a sodium salt thereof.

7. The aqueous oral amlodipine preparation of any of claims 1 to 6, further comprising 15 - 45 wt% of a sweetener.

8. The aqueous oral amlodipine preparation of any of claims 1 to 6, wherein the sweetener is sugar and/or sugar alcohol.

9. The aqueous oral amlodipine preparation of any of claims 1 to 7, which is a liquid.

10. The aqueous oral amlodipine preparation of any of claims 1 to 7, further comprising 0.1 - 3 wt% of a gelling agent and 0.2 - 5 wt% of a fine powder solid.

11. The aqueous oral amlodipine preparation of claim 10, further comprising 0.1 - 2 wt% of a gelling regulator.

12. The aqueous oral amlodipine preparation of claim 11, wherein the gelling regulator is potassium chloride.

13. The aqueous oral amlodipine preparation of any of claims 10 to 12, wherein the gelling agent is carageenan.

14. The aqueous oral amlodipine preparation of claim 10, which is a jelly preparation.

15. A jelly preparation of amlodipine of claim 10, having a composition comprising the following and a liquid property of pH 5 - 7:
a) 0.1 - 1 wt% of amlodipine,
b) sodium lauryl sulfate as the anionic surfactant having a sulfuric acid group or a sulfonic acid group,
c) carageenan as the gelling agent,
d) potassium chloride as the gelling regulator,
e) crystalline cellulose and light anhydrous silicic acid as the fine powder solid,
f) sorbitol as the sweetener.

16. A jelly preparation of amlodipine of claim 10, having a composition comprising the following and a liquid property of pH 5 - 7:
a) 0.1 - 1 wt% of amlodipine,
b) 0.2 - 2.0 wt% of sodium lauryl sulfate,
c) 0.5 - 2 wt% of carageenan,
d) 0.2 - 1 wt% of potassium chloride,
e) 0.5 - 2 wt% of crystalline cellulose and light anhydrous silicic acid,
f) 20 - 45 wt% of sorbitol.

17. The jelly preparation of claim 15 or 16, further comprising 0.05 - 2.0 wt% of a nonionic surfactant.

18. The jelly preparation of any of claims 15 to 17, wherein the nonionic surfactant is a sucrose ester of fatty acid.
